# EUROPEAN PATENT APPLICATION

(11) **EP 4 132 224 A1**
(43) Date of publication of application: **08.02.2023**
(21) Application number: 21189677.4
(22) Date of filing: 04.08.2021
(51) Int. Cl.: H05B 45/20, H05B 47/105, A61N 5/06

(54) **ADJUSTING LIGHTING CONDITIONS**

(71) Applicant: Koninklijke Philips N.V., 5656 AG Eindhoven (NL)
(72) Inventor: Palero, Jonathan Alambra, 5656 AE Eindhoven (NL); BENNIK, Jan, 5656 AE Eindhoven (NL); Varghese, Babu, 5656 AE Eindhoven (NL); Bourquin, Yannyk Parulian Julian, 5656 AE Eindhoven (NL); WANG, Lu, 5656 AE Eindhoven (NL); BISSCHOP, Oedilius Johannes, 5656 AE Eindhoven (NL)
(74) Representative: Philips Intellectual Property & Standards

(57) **Abstract**

According to an aspect, there is provided a computer-implemented method (100) for adjusting lighting conditions, the method comprising: receiving (102) device usage data relating to the use of a personal care device by a subject during a usage event; estimating (104), based on the device usage data, a current emotional state of the subject; receiving (106) an indication of a target emotional state of the subject; determining (108), based on the target emotional state of the subject, a lighting parameter to be applied to a light source to assist the subject in reaching the target emotional state; and operating (110) a light source to emit light according to the determined lighting parameter.

## Description

### FIELD OF THE INVENTION

The invention relates to adjusting lighting conditions and, more particularly, to adjusting lighting conditions based on an emotional state of a person while using a personal care device.

### BACKGROUND OF THE INVENTION

When a person performs a personal care activity, such as shaving, or performing some other treatment in respect of their body, various factors may affect the performance of the activity. In particular, some factors may cause the person (e.g. a user of a personal care device) to perform the activity more effectively or less effectively.

An example of one factor that may affect the ability of a subject to perform a personal care activity to a high standard is the mood or emotional state of the person. For example, a person who is in a bad mood or a negative emotional state may not perform a personal care activity particularly well or effectively, while a person who is in a good or a positive high emotional state may perform a personal care activity much better or more effectively. Moreover, misuse of a personal care device while the subject is in a low or negative emotional state might have consequences in terms of safety, for example leading to skin damage, skin irritation or bruising.

If a person has a bad experience when performing a personal care activity (e.g. if the performance of the activity or treatment fails to provide the expected or anticipated results for the person), then the person may reduce the amount that he or she spends performing that activity in the future. This in turn could lead to the person growing to dislike the personal care activity. There is, therefore, a desire for a method of enhancing or maintaining a person's mood or emotional state during the performance of a personal care activity.

### SUMMARY OF THE INVENTION

The performance of a personal care activity by a person may be more effective if the person is in a good mood or in a positive emotional state. The inventors of the present disclosure have recognised that one way of influencing a person's emotional state is through the use of different ambient light conditions. It has been recognised that humans respond differently to different colors of light (e.g. light of different wavelengths) and, therefore, manipulating ambient light conditions near to a person can affect that person's mood. For example, some colors of light may energise a person while other colors of light may relax a person. Similarly, some colors of light are known to improve a person's mood while other colors are known to invoke feelings of sadness in a person.

In the present disclosure, knowledge of the effects of different lighting conditions on a person's emotional state is used to influence the emotional state of a user of a personal care device. Details of the user's current emotional state are determined, along with details of a target emotional state that should be achieved. Lighting conditions may then be selected in order to influence a transition from the user's current emotional state to a target/intended emotional state.

According to a first specific aspect, there is provided a computer-implemented method for adjusting lighting conditions, the method comprising: receiving device usage data relating to the use of a personal care device by a subject during a usage event; estimating, based on the device usage data, a current emotional state of the subject; receiving an indication of a target emotional state of the subject; determining, based on the target emotional state of the subject, a lighting parameter to be applied to a light source to assist the subject in reaching the target emotional state; and operating a light source to emit light according to the determined lighting parameter.

The device usage data may comprise one or more of i) an indication of the nature of the personal care device being used by the subject; ii) an indication of at least one of: a day of the device usage event, a date of the device usage event, a time of day of the device usage event, a geographic location of the device usage event, a room in a house in which the device usage event occurs, and a device within a defined vicinity of the personal care device during the device usage event; iii) an operating parameter of the personal care device during the device usage event; and iv) sensor data acquired using a sensor associated with the personal care device during the device usage event.

In some embodiments, the sensor data comprises data indicative of one or more of: i) a physiological parameter associated with skin or hair of the subject; a physical parameter associated with skin or hair of the subject; a chemical parameter associated with skin or hair of the subject; and an optical parameter associated with skin or hair of the subject; ii) an operating parameter of the personal care device during the device usage event; and iii) an interaction parameter indicative of an interaction between the personal care device and the subject during the device usage event.

Estimating the current emotional state of the subject may, in some embodiments, comprise consulting a first look-up table relating device usage data to emotional states.

In some embodiments, receiving an indication of the target emotional state of the subject may comprise consulting a second look-up table relating target emotional states to current emotional states.

Determining the lighting parameter may comprise consulting a third look-up table relating lighting parameters to target emotional states.

In some embodiments, the method may further comprise receiving a first user input indicative of an effect of one or more lighting parameters on the emotional state of the subject; and updating the third look-up table based on the first user input.

The method may further comprise receiving subject-specific data indicative of one or more of: lighting preferences of the subject; and personal data of the subject; wherein at least one of estimating the current emotional state of the subject and determining a lighting parameter to be applied is based on the received subject-specific data.

The method may, in some embodiments, further comprise receiving a second user input indicating an emotional state of the subject; wherein estimating the current emotional state of the subject is further based on the received second user input.

In some embodiments, the method may further comprise determining, based on the target emotional state of the subject, a heating parameter to be applied to a heat source in order to assist the subject in reaching the target emotional state; and operating a heat source to generate heat according to the determined heating parameter.

According to a second specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform a method as disclosed herein.

According to a third specific aspect, there is provided a system for adjusting lighting conditions, the system comprising: a personal care device; a light source; and a processing unit in communication with the personal care device and the light source and configured to: receive device usage data relating to the use of the personal care device by a subject during a usage event; estimate, based on the device usage data, a current emotional state of the subject; receive an indication of a target emotional state of the subject; determine, based on the target emotional state of the subject, a lighting parameter to be applied to the light source to assist the subject in reaching the target emotional state; and operate the light source to emit light according to the determined lighting parameter.

The system may, in some embodiments, further comprise a heat generation unit configured to direct heat towards the subject; wherein the processing unit is configured to: operate the heat generation unit to generate heat to be directed towards the subject based on the target emotional state.

In some embodiments, the method may further comprise an imaging unit configured to capture at least one image of at least part of the subject; wherein the processing unit is configured to: estimate a current emotional state of the subject based on the at least one image.

The system may further comprise a user interface configured to receive an input from the subject; wherein the processing unit is configured to: receive an indication of a target emotional state of the subject via a user input provided using the user interface.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a flowchart of an example of a method for adjusting lighting conditions;
Fig. 2 is a flowchart of a further example of a method for adjusting lighting conditions;
Fig. 3 is a schematic illustration of an example of a computer-readable medium in communication with a processor;
Fig. 4 is a schematic illustration of an example of a system for adjusting lighting conditions; and
Fig. 5 is a schematic illustration of a further example of a system for adjusting lighting conditions.

### DETAILED DESCRIPTION OF THE EMBODIMENTS

Various embodiments of the present disclosure provide a mechanism by which a person's emotional state may be influenced using light. More specifically, the emotional state of a user of a personal care device may be influenced (e.g. enhanced or otherwise modified) by adjusting a parameter of a light source that provides ambient light to the user, in order to cause a transition from the users current emotional state to a target emotional state.

Ambient light conditions may be controlled in various situations. For example, the rear housing of a television set may be provided with light-emitting diode (LED) modules to generate backlighting to match or complement images displayed on the screen.

Although ambient lighting that is provided television has been shown to reduce viewer fatigue and improve realism and depth of experience, ambient lighting in general may be used to influence a person's emotions, and may even affect their performance at a particular task (e.g. performing a personal care activity). In a study, six colors (vivid red, vivid blue, vivid yellow, pale red, pale blue, and pale yellow) were manipulated in a simulated study environment to determine their effects on university students' learning performance, emotions, and heart rate. The results showed that, although participants assessed the situation as relaxed, calm, and pleasant in the pale color conditions, reading scores were significantly higher in the vivid color conditions. Heart rates were significantly affected by hue; they increased in the red and yellow conditions. In addition, the results suggested that, regardless of the degree of whiteness, the hue had a significant impact on participants' emotions; blue increased relaxation and calmness feelings of participants compared to the other colors.

Based on color psychology, colors in the red area of the color spectrum (which may be referred to as warm colors and include red, orange, and yellow) evoke emotions ranging from feelings of warmth and comfort to feelings of anger and hostility, while colors on the blue side of the spectrum (which may be referred to as cool colors and include blue, purple, and green) are often described as calm, but can also call to mind feelings of sadness or indifference.

In the lighting industry, color is frequently used to bring a bright, vibrant feel to an environment. Sports games, holidays, and raising awareness for a cause are just a few examples of where color comes into play with lighting. For baby products, the color white is often used, because white symbolizes the beginning of something, innocence, purity, safety, and cleanliness, words that are often associated with babies and new life. Therefore, advertisements with baby-related products draw customers in with the subtle use of the color white, in turn putting all these words in the minds of the viewers.

Just as the color white can be considered to symbolize particular feelings, the color blue is also considered to invoke feelings of calmness, faith, and trust. Blue lighting can affect the human body in a variety of ways. Many findings show that the human eye has photoreceptors that are sensitive to blue light, which has an effect on circadian rhythm. Because of this, blue light is good when waking up in the morning and helping a person readjust their circadian rhythm when travelling, to reduce the effects of jetlag. Blue LED lighting can also be used to increase blood flow, as the skin is also sensitive to the color blue, and can ultimately remove pain in the body and promote healing.

Green is also considered to be a calming color, conveying feelings of hope, soothing and healing. The color green is the most visible and sensitive color to the human eye. Green lighting is sometimes used in operating rooms, as it reduces glare and complements the red color of haemoglobin in blood, to create a more visible environment for surgeons. Green light can also enhance learning and concentration, so is sometimes used in classrooms.

Yellow is considered to be a warming color, which can evoke feelings of happiness or joy, and can improve muscle energy and mental activity. The color yellow is also effective at catching attention. In hospitals, warm tones of yellow lighting are used to create a relaxed and cozy atmosphere, which can help patients get to sleep at night.

Orange is a welcoming color, creating a friendly atmosphere. The color orange is sometimes associated with friendship, endurance, enthusiasm, and creativity. Orange lighting is considered good for bringing home a newborn baby, making them feel at home, welcome and comfortable, particularly since red, orange, and yellow are some of the first colors a human baby can see. It can also stimulate appetite in young people, which is why orange is associated with healthy foods. The color orange can also increase the flow of oxygen to the brain, therefore triggering increased mental activity.

Therefore, different colors and, particularly, different colors of light, may influence of changes in a person's mood, feelings, behaviour or emotional state. Similarly, different colors or different colors of light may affect how a person performs a particular activity. These principles are used in the present disclosure to influence a person's emotional state while they are performing a personal care activity, so that the user has a more positive experience while using a personal care device and, more importantly, so that the user performs the personal care activity in the best (e.g. most effective) way.

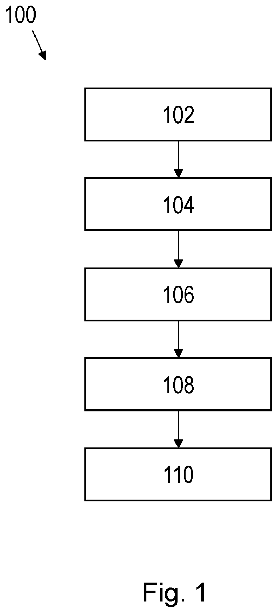

According to a first aspect, the present invention relates to a method. Referring to the drawings, Fig. 1 is a flowchart of an example of a method 100. The method 100 may comprise a computer-implemented method for adjusting lighting conditions. The method 100 comprises, at step 102, receiving device usage data relating to the use of a personal care device by a subject during a usage event. According to the present disclosure, a personal care device, may comprise any device used to perform a personal care activity. For example, a personal care device may comprise, but is not limited to, a shaving device, a hair cutting device, and epilation device, an intense-pulsed laser (IPL) device, a skin treatment device, a power toothbrush, an interactive mirror, a weighing scale, or the like. In the context of the present disclosure, the term "usage event" is intended to refer to an event or duration during which the personal care device is used, for example to perform a treatment or obtain a measurement. The term "usage event" may also be referred to as a "session" or "treatment session". For example, the period from the point at which a subject switches on a shaving device, to the point at which they switch off the shaving device after shaving may be considered to be a usage event.

At step 104, the method 100 comprises estimating, based on the device usage data, a current emotional state of the subject. Thus, the device usage data can be used to estimate how the user of the personal care device (e.g. the subject) may be feeling.

During the usage event, device usage data relating to the manner in which the personal care device is used, is received. The device usage data may comprise any data relating to the usage of the personal care device and may, in some embodiments, comprise one or more of the types of data discussed below.

In one example, the device usage data may comprise an indication of the nature of the personal care device being used by the subject. For example, the device usage data may include a tag or identifier that enables a determination to be made of the type of device being used. In some examples, the personal care device itself may generate a signal including data that can be used to identify the personal care device. In other examples, for example where a camera is available (e.g. a camera of a smart phone or a camera of an interactive mirror), an image of the personal care device may be captured and used to determine the nature of the personal care device. In other examples, the subject (e.g. a user of the personal care device) may provide, via a user input, an indication of the nature of the personal care device being used. The type of personal care device being used during the personal care activity can give an insight as to the emotional state of the subject because some personal care devices may be associated with a more calm or relaxed activity (e.g. epilation using an IPL device), while other personal care devices may be associated with a less relaxed activity (e.g. shaving).

In another example, the device usage data may comprise an indication of at least one of: a day of the device usage event, a date of the device usage event, a time of day of the device usage event, a geographic location of the device usage event, a room in a house in which the device usage event occurs, and a device within a defined vicinity of the personal care device during the device usage event. The day and/or date of the device usage event may give an insight into how relaxed the subject is. For example, a subject to performs a personal care activity at a weekend might be more relaxed than a subject performing a personal care activity on a weekday when they have to work. Similarly, the time of day of the device usage event can be used to determine whether the subject is feeling rushed (e.g. if the personal care activity is being performed before work in the morning) or more relaxed (e.g. if the personal care activity is being performed at night time, before bed). The geographical location at which the device usage event takes place can give an insight into the emotional state of the subject: a user performing a personal care activity at home is likely to be more relaxed than a user performing a personal care activity in a vehicle on their way to work for example. Similarly, within the subject's house, a personal care activity performed in a bathroom environment may be more relaxed than one performed in the kitchen, perhaps a while performing some other activity. If it is determined that the personal care device is being used near to an interactive mirror, then it may be determined that the subject is performing the personal care activity in a relaxed, controlled manner, while if it is determined that the personal care device is not near to (e.g. within a defined vicinity of) another device (e.g. another personal care device), then it may be assumed that the subject is less relaxed. To aid this determination, personal care devices may interact with one another or with a central control unit, for example, to enable their relative positions or relative separation to be determined.

In another example, the device usage data may comprise an operating parameter of the personal care device during the device usage event. For example, if it is determined that a shaving device is set to its maximum speed for the duration of the device usage event, then it may be assumed that the subject is in a rush to perform personal care activity and, therefore, that the subject is somewhat stressed. However, a subject who shaves with the shaving device set to a slower speed may be considered to be more relaxed. Other operating parameters of the personal care device, such as wavelength settings in light-emitting devices, and heat settings in heat-generating devices, may also be used to estimate the subject's emotional state.

In another example, the device usage data may comprise sensor data acquired using a sensor associated with the personal care device during the device usage event. Data from one or more sources may be combined to form the sensor data and/or the device usage data. In some embodiments, the sensor data may comprise data indicative of a physiological parameter associated with skin or hair of the subject, such as a dryness of the skin or hair of the subject. Sweat on the surface of a subject's skin may, for example, imply that the subject is stressed. The sensor data may comprise data indicative of a physical parameter associated with skin or hair of the subject, such as a temperature or color of the skin or hair. Redness of the skin may imply a stressed or angry emotional state of the subject, for example. The sensor data may comprise data indicative of a chemical parameter associated with skin or hair of the subject. For example, certain chemicals present on the hair or skin may be indicative of a particular emotional state of the subject. In other examples, the sensor data may comprise data indicative of an optical parameter associated with skin or hair of the subject, such as an appearance of the skin or hair, capable of providing an indication of the subject's emotional state.

The sensor data may, in some embodiments, comprise data indicative of an operating parameter of the personal care device during the device usage event. As discussed above, operating parameters of a personal care device, such as a temperature setting or a speed setting, may provide an indication of the subject's emotional state. While such settings may be determined from the device itself, in some embodiments, the operating parameters may be determined using one or more sensors associated with the personal care device.

In some embodiments, the sensor data may comprise data indicative of an interaction parameter indicative of an interaction between the personal care device and the subject during the device usage event. For example, a pressure sensor associated with a hair cutting device may be used to determine a pressure applied onto the subject's skin by the hair cutting device. Similarly, and inertial measurement unit (IMU) may be used to determine a speed at which a device (e.g. a shaving device or a power toothbrush) is moved during a personal care activity; a personal care device that is moved rapidly over the course of a personal care activity may be associated with a user who is more stressed or angry than the user who moves the personal care device slowly. In another example, the sensor associated with the personal care device may comprise a camera or a microphone. Such a sensor may record images or sounds during the device usage event, and processing of the images or sounds may be used to estimate the emotional state of the subject. The sensors discussed above may generate data indicative of an interaction between the personal care device and the subject during the device usage event.

The device usage data acquired during the device usage event may be provided to a processing unit, which is configured to estimate the emotional state of the subject. In some examples, estimating the current emotional state of the subject may comprise consulting a first look-up table relating device usage data to emotional states. For example, any of the types of device usage data discussed above may be associated with one or more emotional states, and these associations may be stored in the first look-up table or in a database. The device usage data may be compared to various thresholds (e.g. stored in a database or a look-up table, such as the first look-up table) to determine a likely emotional state of the subject based on the received device usage data. One or more algorithms may be used to combine the different types of device usage data in order to improve the estimation of the emotional state of the subject. For example, a subject who uses a shaving device set to a relatively slow setting, who moves the shaving device slowly over his face to perform a shaving event in the bathroom of his house on a Sunday evening may be considered to be in a very calm and relaxed state. Conversely, a subject who uses a shaving device set to a high speed setting, and who moves this shaving device rapidly over his face during a shaving event performed in the kitchen of his house on a Monday morning may be considered to be in a stressed emotional state.

The method 100 comprises, at step 106, receiving an indication of a target emotional state of the subject. While the current emotional state of the subject may be considered to be the initial starting emotional state of the subject (e.g. beginning of the device usage event), the target emotional state may be considered to be the emotional state that it is intended that the subject reaches. In some examples, the target emotional state may be the same as the current emotional state. For example, if it is estimated that the subject is currently happy, then it may be intended that the subject stays happy. In some examples, an indication of a target emotional state of the subject may be received via a user input provided by the subject. For example, the subject may indicate via a user interface on their smart phone or on an interactive mirror that they would like to more relaxed that they would like to feel more energised.

In some examples, receiving an indication of the target emotional state of the subject comprises consulting a second look-up table relating target emotional states to current emotional states. For example, in the event that the subject has not provided an indication of their intended target emotional state, the second look-up table may provide a standard or predefined association between current emotional states and target emotional states. This may, for example, assume that a subject who is feeling low or stressed is likely to want to have their mood lifted or to feel less stressed. In other examples, the target emotional state may be determined using a previously-indicated target emotional state for the subject. For example, the subject may have indicated previously that they generally like to feel relaxed and, as such, this may be used as a default position for the subject target emotional state. In some embodiments, target emotional states of a population of people may be used to determine the target emotional state for the subject. For example, an analysis may be performed of the target emotional states of a population of people who have similar attributes to the subject (e.g. who have profiles that match or are similar to that of the subject), and the target emotional states of those people may be used to determine the target emotional state of the subject. In one example, it may be assumed that, first thing in the morning, a particular demographic of people might desire to be in a similar emotional state.

At step 108, the method 100 comprises determining, based on the target emotional state of the subject, a lighting parameter to be applied to a light source to assist the subject in reaching the target emotional state. The lighting parameter may comprise a parameter (e.g. an operating parameter) or setting of the light source that controls an aspect of the light emitted by the light source. Thus, in some embodiments, the lighting parameter may comprise at least one of a wavelength of light to be emitted; a warmth of light to be emitted; and an intensity of light to be emitted. In other examples, the lighting parameter may comprise a power or intensity of light, a frequency of light and/or a color of light (e.g. using one or more filters). In other examples, other lighting parameters may be applied.

The selection of the lighting parameter to be applied to the light source is made with the intention of causing the subject to reach the target emotional state. For example, if it is determined that the subject is currently in a low or sad emotional state, and the target emotional state is one of happiness, then it may be determined that the lighting parameter to be applied to the light source is one that causes the light source to emit light associated with feelings of happiness and joy, such as yellow light. In some embodiments, determining the lighting parameter may comprise consulting a third look-up table relating lighting parameters to target emotional states. For example, as noted in the above example, a target emotional state of happiness may be associated in the look-up table with a particular lighting parameter that, if applied, would cause the light source to emit yellow light (e.g. a particular wavelength setting associated with yellow light

As noted above, the target emotional state may, in some examples, be the same as the current emotional state. For example, the subject may already be in a positive, happy mood and the target emotional state may also be one of happiness. In such examples, it is not necessary to change the emotional state of the subject, so the lighting parameter to be applied to the light source may be the lighting parameter that is currently being applied to the light source, or may be a parameter that causes light to be emitted that maintains the subject's current emotional state.

In some embodiments, the determination of a lighting parameter to be applied to the light source may be based further on the current emotional state of the subject. Thus, rather than basing the determination of the lighting parameter solely on the target emotional state, the current emotional state of the subject may also be taken into account. In such examples, there may exist particular lighting parameters that are known to be particularly effective at transitioning a user between particular current and target emotional states. Associations between particular current and target emotional states, and effective lighting parameters for enabling transitions between those emotional state may be stored in one or more look-up tables, such as the third look-up table.

The method 100 may comprise, at step 110, operating a light source to emit light according to the determined lighting parameter. For example, the determined lighting parameter may be applied to the light source. This may cause the light source to emit light that helps the subject to reach the target emotional state. In some embodiments, operating the light source, or applying the determined lighting parameter to the light source may be done in such a way that a transition from light currently being emitted by the light source into light emitted according to the determined lighting parameter is smooth or gradual. Changing the lighting conditions in a slow, gradual way may be less apparent or noticeable to the subject of the subject is less aware of the intended change that is to occur in their emotional state.

Applying lighting parameters to a light source in order to cause the light source to emit light can help change a subject's emotional state. Not only can this help to improve the mood of a person, but it can also have a positive effect on the way in which the subject performs a personal care activity using the personal care device. For example, treatment (e.g. hair removal) using an intense pulsed light (IPL) device should be performed slowly and carefully, and the device should be moved incrementally over the area of the body to be treated. If the device is moved too quickly, then patches of skin may be missed, and hair may not be removed effectively. Therefore, it is desirable that the subject using the IPL device is in a calm and relaxed emotional state. The method 100 can, therefore, help to create an environment, using ambient lighting conditions, that help to relax the subject, thereby making it more likely that the subject performs the treatment slowly and effectively. This can result not only in a treatment being performed as intended, but can also have benefits in terms of safety. For example, operating some personal care devices while in an agitated or angry emotional state may misuse of the device and could ultimately lead to the occurrence of accidents. Similarly, using a personal care device while in an angry emotional state could lead to damage of the personal care device (e.g. if the device is not used properly or in accordance with the intended operating conditions. A user in a calmer emotional state is more likely to use the personal care device in an intended manner, which is less likely to result in the device being damaged.

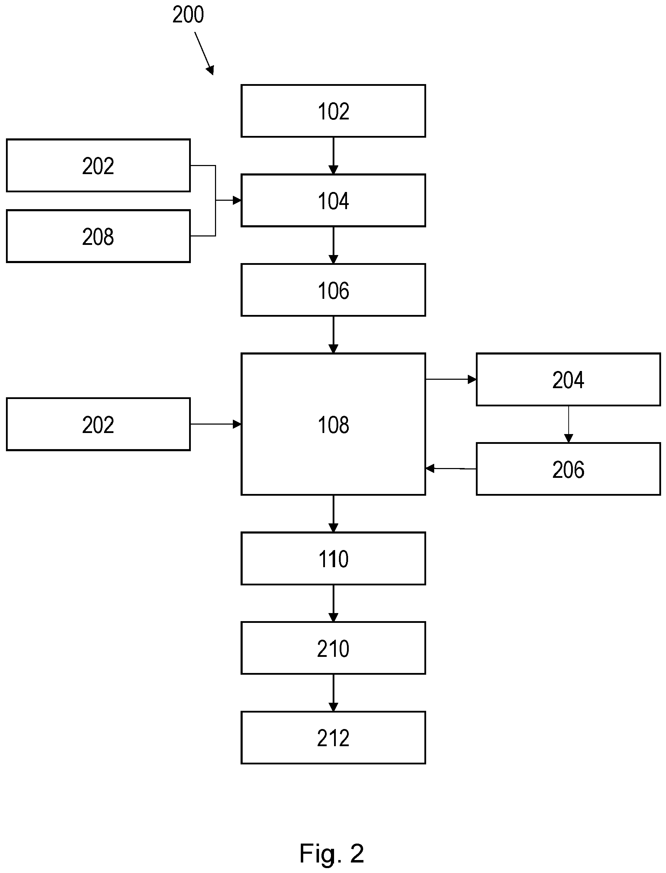

Fig. 2 is a flowchart of a further example of a method 200, which may comprise a computer-implemented method for adjusting lighting conditions. The method 200 may comprise one or more steps of the method 100 discussed above. At step 202, the method 200 may further comprise receiving subject-specific data indicative of one or more of lighting preferences of the subject and/or personal data of the subject. The subject-specific data may comprise any data relating to the subject that provides an indication of the subject's preferences with regard to lighting conditions (e.g. an indication of particular colors of light that have a particular effect on the subject's emotional states). The subject-specific data may, for example, be provided by a subject via a user interface. In some examples, a user profile may store subject-specific data and/or preferences associated with a particular subject, and used to obtain the subject-specific data.

The step of estimating (step 104) the current emotional state of the subject and/or the step of determining (step 108) a lighting parameter to be applied may be based on the received subject-specific data. For example, the subject-specific data may include an indication of the subject's current emotional state or an indication of a preferred lighting condition of the subject.

At step 204, the method 200 may further comprise receiving a first user input indicative of an effect of one or more lighting parameters on the emotional state of the subject. For example, the subject may provide a first user input indicating that a particular lighting parameter (e.g. a particular color of light) makes them feel angry or sad. The input provided by the subject can be used to update the look-up tables. For example, the method 200 may further comprise, at step 206, updating the third look-up table based on the first user input. In this way, the look-up tables consulted during the methods 100, 200 can be personalised to various subjects, reducing the chance that applying a particular lighting parameter for a subject has an undesired effect (e.g. making them sad when the target emotional state is happy).

The method 200 may further comprise, at step 208, receiving a second user input indicating an emotional state of the subject. For example, the subject may provide an indication by selecting from a list of emotional states displayed on a screen of a smart phone or an interactive mirror. The subject's input (i.e. the second user input) may be used in combination with the device usage data to determine the subject's current emotional state. Thus, estimating (step 104) the current emotional state of the subject may be further based on the received second user input.

In addition to causing a particular lighting condition to be provided to the subject in order to invoke a target emotional state, in one embodiment, the application of heat may also be used to help reach the target emotional state. Thus, in some embodiments, the personal care device, or another device, may be provided with heat generation means heat emitting means, configured to provide heat to the subject in order to help the subject to reach the target emotional state. Thus, in some embodiments, the method 200 may further comprise, at step 210, determining, based on the target emotional state of the subject, a heating parameter to be applied to a heat source in order to assist the subject in reaching the target emotional state. In some embodiments, determining the heating parameter (step 210) may be further based on the current emotional state of the subject. In an example, if it is determined that the target emotional state of the subject is cosiness, then the method 200 may determine at step 210 that the heat source is to apply heat at a relatively high temperature, causing the subject to warm up and helping the subject to reach the target emotional state. Thus, at step 212, the method 200 may further comprise operating a heat source to generate heat according to the determined heating parameter. The heat may be provided in the form of hot air and/or radiation (e.g. infrared light). In some embodiments, the light source may comprise an infrared light source, and the lighting parameter may comprise a parameter that causes infrared radiation to be emitted.

Steps of the methods 100, 200 discussed above may be performed using one or more processors or processing units. Such processors may form part of a device, such as the personal care device, a smart phone, a smart watch, a tablet computer, a laptop computer, an interactive mirror, or the like. In some embodiments, the processing may be performed remotely from the subject, for example using a cloud-computing environment. Thus, the processor may form part of a remotely-located server, for example.

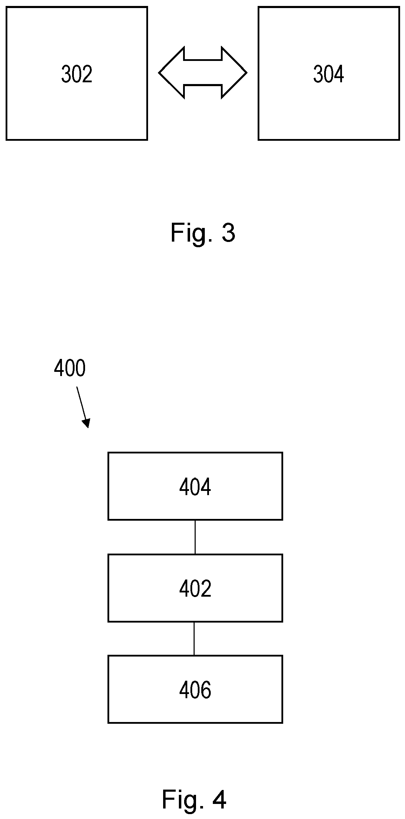

According to a second aspect, the present invention relates to a computer program product. Fig. 3 is a schematic illustration of an example of a processor 302 in communication with a computer-readable medium 304. According to an embodiment, a computer program product comprises a non-transitory computer-readable medium 304, the computer-readable medium having computer-readable code embodied therein, the computer-readable code being configured such that, on execution by a suitable computer or processor 302, the computer or processor is caused to perform steps of the methods 100, 200 disclosed herein.

According to third aspect, the present invention relates to a system. Fig. 4 is a schematic illustration of an example of a system 400, which may be considered to be a system for adjusting lighting conditions. The system 400 comprises a processing unit 402, a personal care device 404 and a light source 406. The processing unit 402 is in communication with the personal care device 404 and the light source 406 and is configured to perform one or more steps of the methods 100, 200 discussed herein. Specifically, the processing unit 402 is configured to receive device usage data relating to the use of the personal care device by a subject during a usage event, estimate, based on the device usage data, a current emotional state of the subject, receive an indication of a target emotional state of the subject, determine, based on the target emotional state of the subject, a lighting parameter to be applied to the light source to assist the subject in reaching the target emotional state, and operate the light source to emit light according to the determined lighting parameter. Components of the system 400 may be located in a single device or located remotely from one another in two or more devices.

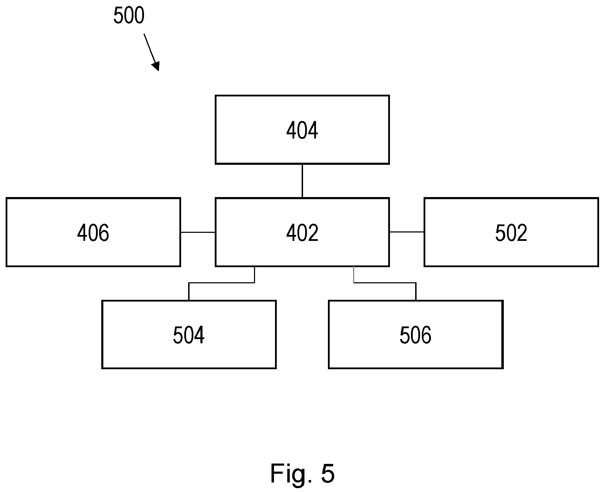

Fig. 5 is a schematic illustration of a further example of a system 500, such as a system for adjusting lighting conditions. The system 500 comprises components of the system 400 disclosed above. The system 500 may further comprise a heat generation unit 502 configured to direct heat towards the subject. The heat generation unit 502 may, for example, form part of the personal care device 404, or it may comprise a separate component remote from the personal care device. The processing unit 402 may be configured to operate the heat generation unit 502 to generate heat to be directed towards the subject based on the target emotional state. As discussed above, the heat generation unit 502 may be configured to generate and direct high-temperature heat towards the subject if the target emotional state of the subject is cosiness or relaxed.

The system 500 may further comprise an imaging unit 504 configured to capture at least one image of at least part of the subject. The imaging unit 504 may, for example, comprise a camera in a smart phone or tablet computer, or a camera in an interactive mirror. The imaging unit 504 may capture a single image or multiple images, such as stream of images forming a video. The processing unit 402 may be configured to estimate a current emotional state of the subject based on the at least one image. For example, facial expression analysis may be performed to determine a mood or emotional state of the subject based on the facial expression of the subject. In other examples, a determination of the current emotional state of the subject may be determined by analysing image data available in the captured image, such as a color of the subject's skin (e.g. redness in the subject's face may imply anger).

In some embodiments, the system 500 may comprise a microphone (not shown) recording sound data associated with the subject. The recorded sound data may be used alone or in conjunction with images captured by the imaging unit 504 to help determine the current emotional state of the subject.

The system 500 may further comprise a user interface 506 configured to receive an input from the subject. The user interface 506 may comprise one or more switches, one or more buttons, a keypad, a keyboard, a mouse, a mouse wheel, a touch screen or an application (for example, on a tablet or smartphone), or any other user interface, or combination of user interfaces suitable for enabling the subject to provide a user input. The user interface 506 may be used by the subject to indicate their current emotional state and/or their target emotional state, along with other data that may be used for example to update one or more of the look-up tables. The processing unit 402 may be configured to receive an indication of a target emotional state of the subject via a user input provided using the user interface 506.

Embodiments disclosed herein provide a mechanism by which the mood or emotional state of a subject may be influenced through the use of ambient light. Based on an estimate of the subject's current emotional state while performing a personal care activity, and the determination of a target emotional state that the subject should or would like to reach, appropriate lighting conditions may be determined and lighting parameters may be applied in order to create the desired lighting conditions. By changing or improving the subject's mood or emotional state, the subject may perform the personal care activity to a higher standard, more effectively and/or more safely. Moreover, by improving the way that the subject uses the personal care device (e.g. by creating a calmer environment in which to use the device) the personal care device is likely to last longer, and is less likely to be damaged. The processor 302, 402 can comprise one or more processors, processing units, multi-core processors or modules that are configured or programmed to control the system 400, 500 in the manner described herein. In particular implementations, the processor 302, 402 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100) for adjusting lighting conditions, the method comprising:
receiving (102) device usage data relating to the use of a personal care device by a subject during a usage event;
estimating (104), based on the device usage data, a current emotional state of the subject;
receiving (106) an indication of a target emotional state of the subject;
determining (108), based on the target emotional state of the subject, a lighting parameter to be applied to a light source to assist the subject in reaching the target emotional state; and
operating (110) a light source to emit light according to the determined lighting parameter.

2. A computer-implemented method (100) according to claim 1, wherein the device usage data comprises one or more of:
i) an indication of the nature of the personal care device being used by the subject;
ii) an indication of at least one of: a day of the device usage event, a date of the device usage event, a time of day of the device usage event, a geographic location of the device usage event, a room in a house in which the device usage event occurs, and a device within a defined vicinity of the personal care device during the device usage event;
iii) an operating parameter of the personal care device during the device usage event; and
iv) sensor data acquired using a sensor associated with the personal care device during the device usage event.

3. A computer-implemented method (100) according to claim 2, wherein the sensor data comprises data indicative of one or more of:
i) a physiological parameter associated with skin or hair of the subject; a physical parameter associated with skin or hair of the subject; a chemical parameter associated with skin or hair of the subject; and an optical parameter associated with skin or hair of the subject;
ii) an operating parameter of the personal care device during the device usage event; and
iii) an interaction parameter indicative of an interaction between the personal care device and the subject during the device usage event.

4. A computer-implemented method (100) according to any of the preceding claims, wherein estimating (104) the current emotional state of the subject comprises consulting a first look-up table relating device usage data to emotional states.

5. A computer-implemented method (100) according to any of the preceding claims, wherein receiving (106) an indication of the target emotional state of the subject comprises consulting a second look-up table relating target emotional states to current emotional states.

6. A computer-implemented method (100) according to any of the preceding claims, wherein determining (108) the lighting parameter comprises consulting a third look-up table relating lighting parameters to target emotional states.

7. A computer-implemented method (100, 200) according to claim 6, further comprising:
receiving (204) a first user input indicative of an effect of one or more lighting parameters on the emotional state of the subject; and
updating (206) the third look-up table based on the first user input.

8. A computer-implemented method (100, 200) according to any of the preceding claims, further comprising:
receiving (202) subject-specific data indicative of one or more of: lighting preferences of the subject; and personal data of the subject;
wherein at least one of estimating (104) the current emotional state of the subject and determining (108) a lighting parameter to be applied is based on the received subject-specific data.

9. A computer-implemented method (100, 200) according to any of the preceding claims, further comprising:
receiving (208) a second user input indicating an emotional state of the subject;
wherein estimating (104) the current emotional state of the subject is further based on the received second user input.

10. A computer-implemented method (100, 200) according to any of the preceding claims, further comprising:
determining (210), based on the target emotional state of the subject, a heating parameter to be applied to a heat source in order to assist the subject in reaching the target emotional state; and
operating (212) a heat source to generate heat according to the determined heating parameter.

11. A computer program product comprising a non-transitory computer-readable medium (304), the computer-readable medium having computer-readable code embodied therein, the computer-readable code being configured such that, on execution by a suitable computer or processor (302), the computer or processor is caused to perform the method of any of the preceding claims.

12. A system (400) for adjusting lighting conditions, the system comprising:
a personal care device (404);
a light source (406); and
a processing unit (402) in communication with the personal care device and the light source and configured to:
receive device usage data relating to the use of the personal care device by a subject during a usage event;
estimate, based on the device usage data, a current emotional state of the subj ect;
receive an indication of a target emotional state of the subject;
determine, based on the target emotional state of the subject, a lighting parameter to be applied to the light source to assist the subject in reaching the target emotional state; and
operate the light source to emit light according to the determined lighting parameter.

13. A system (400, 500) according to claim 12, further comprising:
a heat generation unit (502) configured to direct heat towards the subject; wherein the processing unit (402) is configured to:
operate the heat generation unit to generate heat to be directed towards the subject based on the target emotional state.

14. A system (400, 500) according to claim 12 or claim 13, further comprising:
an imaging unit (504) configured to capture at least one image of at least part of the subject; wherein the processing unit (402) is configured to:
estimate a current emotional state of the subject based on the at least one image.

15. A system (400, 500) according to any of claims 12 to 14, further comprising:
a user interface (506) configured to receive an input from the subject; wherein the processing unit (402) is configured to:
receive an indication of a target emotional state of the subject via a user input provided using the user interface.
